# EUROPEAN PATENT SPECIFICATION

(11) **EP 1 420 743 B1**
(45) Date of publication and mention of the grant of the patent: **19.08.2009**
(21) Application number: 01989283.5
(22) Date of filing: 20.12.2001
(51) Int. Cl.: A61K 31/575, A61K 47/26

(54) **CHOLESTEROL SULFATE AND AMINO SUGAR COMPOSITIONS FOR ENHANCEMENT OF STRATUM CORNEUM FUNCTION**
ZUSAMMENSETZUNGEN VON CHOLESTEROLSULFAT UND AMINOZUCKERN ZUR VERBESSERUNG DER STRATUM CORNEUM FUNKTION
COMPOSITIONS DE SULFATE DE CHOLESTEROL ET DE SUCRE AMINE PERMETTANT D'AMELIORER LA FONCTION DE LA COUCHE CORNEE DE L'EPIDERME

(30) Priority: 31.01.2001 US 773351
(43) Date of publication of application: 26.05.2004
(73) Proprietor: Color Access, Inc., Melville, New York 11747 (US)
(72) Inventor: ZECCHINO, Julius, R., Closter, NJ 07624 (US); MAES, Daniel, H., Huntington, NY 11743 (US)
(74) Representative: Hirsch & Associés
(86) International application number: PCT/US2001/050767
(87) International publication number: WO 2002/060381

(56) References cited:
- WO-A-90/01323
- WO-A1-00/45786
- US-A- 5 650 166
- US-A- 6 150 381

## Description

### FIELD OF THE INVENTION

The present invention relates to cosmetic compositions. More specifically, the invention relates to topical compositions containing a combination of cholesterol sulfate and an amino sugar for the treatment of skin.

### BACKGROUND OF THE INVENTION

The stratum corneum represents the major chemical and physical barrier between the body and the environment. It is formed by a process in the epidermis which involves the transformation of germinative cells into terminally differentiated cells; the process of transformation takes approximately one month, by which time the terminally differentiated cells are shed from the skin surface. The cells at the outermost layer of the skin, which regularly slough off, are replaced by cells that generate originally at the basal layer of the epidermis and rise up to the outer surface as other newer cells are generated at the basal layer. As these cells migrate from the basal layers up to the outer levels of the skin, these cells produce and accumulate keratin, to the point at which there is virtually no cytoplasm remaining; at this point, the cell dies and sheds off of the skin. The shed layer of dead skin reveals a fresh layer of healthy skin, another phalanx of migrating epidermal cells. The thickness of the stratum corneum and epidermis, in general, varies in different regions of the body, and plays a role in the rate of shedding dead skin layers.

The cornified barrier performs a number of functions. As mentioned, a particularly important aspect of its presence is as a physical barrier, between the deeper layers of the skin as well as the internal organs and the environment. Prevention or attenuation of penetration of UV radiation, as well as other harmful stimuli such as free radicals, to the deeper skin layers are examples of the critical aspect of this skin layer. The skin acts as a permeability barrier and therefore, the skin functions to prevent the loss of body water to the external environment. Unfortunately, as with many other skin functions, the capacity of the stratum corneum to cyclically generate new layers of the skin progressively diminishes with age. The stratum corneum is a densely packed structure of intracellular fibrous elements that are hydrophilic and able to trap water. The intercellular space is filled with lipids that provide a diffusion pathway to channel substances with low solubility in water. Thus, the skin barrier of the stratum corneum resembles a brick wall, the corneocytes are the bricks and the intercellular matrix is the mortar. The turnover rate of the stratum corneum is considerably decreased in older individuals, however, and the cornified layer gradually becomes thinner, thereby reducing the efficacy of this physical barrier and making it easier for harmful stimuli such as UV rays to penetrate the skin barrier. This in turn leads to UV-damage to the dermal layers of the skin, degradation of collagen and elastin, and finally, wrinkling and skin atrophy. These are examples of a skin barrier that is compromised and unhealthy. Moreover, the thinning of the stratum corneum can enhance the visibility of wrinkling and atrophy, the cause of which is rooted in the dermis.

To improve the signs of wrinkling and atrophy, exfoliation is a commonly used technique whereby dead skin cells are physically removed or sloughed from the skin surface by human intervention. An exfoliant breaks the bond holding individual squames together and allows them to detach and shed. This technique promotes a healthier and more youthful appearance to the skin. The skin cells are held together by a desmosome bond between corneocytes at the skin surface. The bond delays the desquamation of the skin cells, reinforces the barrier, and prevents water loss. The physical action of removing dead skin cells can be achieved by active agents as well as by manual scrubbing action. Several compounds are known to be useful as exfoliants such as for example, alpha hydroxy acid ("AHA"), beta hydroxy acid ("BHA"), retinoic acid ("retin A"), and enzymes. In addition, it is disclosed in PCT Patent Application No. WO US00/11203, that N-acetyl-D-glucosamine is useful as an exfoliant.

In addition to the exfoliative process that improves the smoothness of the skin surface, the skin cells, before they die and slough off, need to maintain a cohesiveness to support the thickness and firmness of the skin. A thicker stratum corneum helps to prevent or retard the appearance of fine lines and wrinkles. Protease inhibitors are known to prevent the breakage of the desmosome bond between the corneocytes at the skin surface. In addition, cholesterol sulfate is also known to retard desquamation in the stratum corneum of the skin, as disclosed in PCT Publication No. WO00/45786.

Notwithstanding the obvious importance of the stratum corneum in maintaining a healthy youthful appearance of the skin, rehabilitation and maintenance of the dermis has been a major cosmetic focus in preventing the appearance of aging; relatively little attention has been paid to developing a cosmetic means for maintaining a fairly consistent level of stratum corneum function into old age. Producing one cosmetic composition for the skin that achieves a variety of interdependent activities in the skin to maintain the health and beauty of the skin surface (i.e., a healthy skin barrier) is a desirable and beneficial need. Further, the availability of alternative methods to exfoliate different skin types and meet various individual skincare needs of consumers is beneficial. Thus, there is a continued effort to find additional alternative ways of aiding the sloughing ability of the skin and promoting the health of various types of skin. This is, therefore, an object of the present invention.

### Summary of the Invention

The invention relates to a composition for topical application to the skin that is a mixture of an effective amount of an exfoliant and cholesterol sulfate. The exfoliant is an amino sugar selected from the group consisting of N-acetyl-D-glucosamine, N-acetylgalactosamine, and combinations thereof. The cholesterol sulfate is present in the composition in an amount of between about 0.05 to about 5.00 percent and the exfoliant is present in an amount of about 0.1 to about 10.0 percent. The combination of these two components enhances the protective barrier of the skin and repairs the barrier if it has been damaged by chronological aging or other environmental factors. Thus, the present invention also includes the use of an effective amount of the mixture of two components for preparing a composition for applying to the skin for improving or maintaining a healthy skin barrier. Because the skin barrier can be improved or maintained, another method of the present invention is treating or preventing damage to the skin, when the damage is associated with a reduction or a loss of skin barrier function, by topically applying to the skin a mixture of the two components.

### Detailed Description of the Invention

The present invention, in its various embodiments, is predicated on the surprising observation that effective amounts of cholesterol sulfate, which is known to reduce desquamation, and an exfoliant, can be combined in a mixture to improve the quality of the protective barrier of the skin. As mentioned above, cholesterol sulfate enhances the cohesion of the stratum corneum resulting in a more prolonged retention of the layers of the stratum corneum. Further, it has been observed that application of cholesterol sulfate to skin cells results in a distinct, dose-dependent, increase in the thickness of the layers of the stratum corneum. The observation is important for a number of different applications; a particularly significant application is in the maintenance of the texture of older skin. Thus, application of cholesterol sulfate to retard desquamation and maintain stratum corneum thickness treats and maintains older, thinning skin. A thicker stratum corneum aids in preventing or retarding the appearance of fine lines and wrinkles which so frequently characterize thinning skin. At the same time, the enhanced cohesion of the stratum corneum results in an effective strengthening of the protective lipid barrier naturally provided therein. However, the skin surface can become rough and feel tough because dead skin cells at the surface of the skin buildup on the skin surface. As a result, the skin can look dry and flaky because it is unhealthy.

A current and common means of enhancing smoothness of the skin is to encourage exfoliation. However, exfoliation necessarily involves a high rate of turnover of the stratum corneum, and consequent thinning of this layer of the skin. While not an issue in youthful skin, desquamation in older skin can, in some cases, simply exacerbate a problem already established, namely, the natural thinning observed with age. Therefore, it is not known to use an exfoliant in combination with cholesterol sulfate when it is desired to thicken and strengthen the protective barrier of the skin. The present invention of improving or protecting the barrier of the stratum corneum is surprising because it is achieved with a combination of two components having opposing activities. One component acts to desquamate the skin and the other component acts to retard desquamation. However, a balanced nurturing result is achieved with the present invention on the skin barrier of the stratum corneum.

The discovery that two opposing components do not cancel each other out in a composition for the skin was unexpected. In addition, the beneficial effect can be appreciated by individuals of all ages. The stratum corneum represents an important physical barrier between the environment and the deeper skin layers as well as the internal organs. The present invention produces a thicker layer of the stratum corneum while also promoting the cycle of removing dead skin cell layers of the stratum corneum. Therefore, moisture is retained in the skin. The skin is firmer, lines and wrinldes are less apparent, and the skin surface is soft and smooth. Maintaining the health of the skin is a complex and integrated process requiring a delicate balance between promotion of proliferation, support of differentiation, and regulating desquamation. For example, increased proliferation activity of the epidermis is known to be the cause or a factor in skin disorders such as psoriasis, ichthyoses, and essential fatty acid deficiency. While not wishing to be bound by any theory it is believed that the combination of these two components work on different skin surface layers to produce a positive result on the skin barrier function. The ability to achieve these integrated results in one composition containing two components that act in opposite ways has not heretofore been known.

To achieve this effect, one component of the mixture in the present invention is cholesterol sulfate or salts thereof. The salts can be sodium, potassium, magnesium, or other similar salts. Preferably, the salt is potassium cholesterol sulfate. The other component of the mixture is the exfoliant and can be an amino sugar such as N-acetyl-D-glucosamine, N-acetylgalactosamine, or a combination of the two. Preferably, the amino sugar is N-acetyl-D-glucosamine. Although the upper limit is not critical, cholesterol sulfate is effective in the mixture when provided in an amount of from about 0.05 to about 5.00 percent, preferably 0.1 to about 2.0 percent, and most preferably about 0.04 to about 1.00 percent, all by weight of the total composition. The amino sugar is present in the mixture in an amount of about 0.1 to about 10.0 percent, preferably about 0.5 to about 8.0 percent of the weight of the composition; and more preferably about 0.8 to about 2.0 percent of the weight of the composition. Similarly, the upper limit of the amounts of the exfoliant is not critical. As used herein the effective amounts of cholesterol sulfate and the exfoliant means amounts of each of the two components sufficient to maintain a healthy skin barrier, or to enhance or repair the skin barrier at least about 10 percent over a formula without the two components in combination, preferably about 20 percent over the formula without the two components, and more preferably about 50 percent over the formula without the two components. The maintenance of a healthy skin barrier, or the enhancement or repair of the skin barrier can be measured by determining the transepidermal water loss after a barrier challenge, or other standard methods known to one skilled in the art.

The combination of these two components can be applied in any type of cosmetically or pharmaceutically acceptable vehicle for topical application with which the active component is compatible, e.g., a gel, a cream, a lotion, an ointment, a mousse, a spray, a solid stick, a powder, a suspension, a dispersion, and the like. Techniques for formulation of various types of vehicles are well known to those skilled in the art, and can be found, for example, in Chemistry and Technology of the Cosmetics and Toiletries Industry, Williams and Schmitt, eds., Blackie Academic and Professional, Second Edition, 1996, and Remington's Pharmaceutical Sciences, 18th Edition, 1990.

In addition to its use in therapeutic products, the mixture of cholesterol sulfate and amino sugar can also be beneficially added to color cosmetic products. In this regard, effective amounts of the mixture are added to makeup formulations such as foundations, blushes, lipsticks and glosses, eyeliners, eyeshadows, and the like. A particular advantage may be obtained with such formulations, in that the retardation of desquamation may enhance makeup retention on the skin to which it is applied but the sloughing activity softens the skin and reduces the appearance of fine lines and wrinkles.

In all formulations in which enhancement of the protective barrier of the skin is involved, it is preferred that the mixture of cholesterol sulfate and the exfoliant be combined with other components of the naturally occurring skin barrier. In a particularly preferred embodiment, the cholesterol sulfate is combined with at least one fatty acid and cholesterol. Fatty acids may be up to 24 carbon atoms in length. Examples of fatty acids include butyric acid, caproic acid, octanoic acid, decanoic acid, dodecanoic acid, tetradecanoic acid, palmitic acid, stearic acid, linoleic acid and oleic acid. A preferred fatty acid is linoleic acid.

In addition to the two components of the present invention, the compositions can also include one or more ceramides. The ceramides to be employed in the compositions of the invention are sphingolipids, having a sphingosine or related molecule backbone with fatty acids or ω-esterified fatty acids linked to an amino group on the sphingosine, and in some cases, with saccharide moieties linked to the terminal hydroxyl of the sphingosine. In particular, the compositions may contain ω-esterified ceramides or acylceramides, cerebrosides, ω-esterified cerebrosides, or acylglycosyl sphingolipids. Particularly preferred types of ceramides for the present compositions are ceramide III and cerebrosides. Other similar lipids that can be included in the compositions of the present invention are, for example, wheat bran extract, olive extract, wheat germ extract, barley extract, and other similar lipid containing extracts.

In those compositions in which cholesterol sulfate is combined with these lipids, the lipid components each can be used in an amount of from about 0.01 to 1.00 percent, preferably 0.02 to about 0.50 percent, most preferably about 0.02 to about 0.10 percent, all by weight of the total composition. In a particularly preferred embodiment, the cholesterol sulfate, the fatty acid, cholesterol, and the lipid components are premixed before adding to the exfoliant in the composition. It will be understood from the foregoing that the lipid component need not be pure lipid, but rather may be natural extracts containing one or more desirable lipids, and used in amounts consistent with attaining the concentrations recommended above.

In another embodiment of the present invention, the compositions contain sclareolide and white birch. It has been reported in U.S. Patent No. 6,150,381 that sclareol-like and sclareolide-like compounds are useful in treating microbial infections. Sclareol is an important bioactive diterpene obtained from clary sage (*Salvia sclarea Labiatae*.) The clary sage extract is believed to contain about 70 percent sclareol. In addition, another useful species of the genus *Salvia,* is *Salvia officinalis L.* Methods of using *Salvia officinalis* in an external ointment have been disclosed in U.S. Patent No. 5,660,831 for controlling high blood-pressure, circulatory problems, and incomplete cicatrization of wounds. The characteristic constituents of *Salvia officinalis* (Dalmation sage) are believed to be alpha-(about 30 to 40 percent) and beta- thujone (about 10 percent). As used in the present invention, the source of sclareolide can be derived naturally from either species of *Salvia,* or can be synthetically obtained as substantially pure sclareolide. Substantially pure sclareolide contains greater than 70 percent sclareolide. In the composition, sclareolide is effective in an amount of about 0.001 to about 5.000 percent by weight of the total composition.

The use of white birch as a protease inhibitor in combination with a cell differentiation enhancer has been disclosed in copending U.S. Application Serial No. 09/554984. Although the presence of cholesterol sulfate provides the surprising ability to retard desquamation in the presence of the exfoliant, additional retardation of desquamation can be provided cell differentiation enhancers, such as, for example white birch. Other useful protease inhibitors include, but are not limited to, triterpenoid-containing extracts and refined compounds, for example, silver birch bark extract, *Boswellia* extract, bearberry extract, *Centella asiatica* extract, or *Pygeum* (Prunus) *africanum* extract, and individual protease inhibitor compounds that may be present in these extracts, including betulinol (betulin), betulinic acid, boswellic acid, ursolic acid, oleanolic acid, oleanol, asiaticoside, asiatic acid, and madagassic acid; phenolic-containing extracts, such as green tea extracts and apple extracts, and compounds contained therein, such as EGCG, ECG, catechins, phenylpropanoids, and phloretin; protein-based extracts, such as soy protein, or egg protease inhibitors, or other phytosterol sulfates. The preferred protease inhibitor is white birch bark extract. If an additional cell differentiation enhancer is incorporated in the compositions of the present invention, it is present in an amount of about 0.001 to about 1.000 percent by weight of the total composition, and preferably about 0.05 to about 0.5 percent.

The present invention may include applying in addition to the two components of the mixture, other optional components, including, but are not limited to, additional exfoliants, preservatives, fragrances, emollients, antiseptics, antiinflammatories, antibacterials, stabilizers, antioxidants, vitamins, pigments, dyes, humectants, surfactants, and propellants, as well as other classes of materials the presence of which in the compositions may be cosmetically, medicinally, or otherwise desired. Such components can be found in the CTFA International Cosmetics Ingredients Dictionary. Examples of additional exfoliants include but are not limited to chemical exfoliants such as AHAs, for example, lactic acid, or BHAs, for example, salicylic acid, or physical exfoliants such as pumice, polyethylene, walnut shell powder, and the like, or combinations thereof. The amount of additional exfoliants alone or in combination will depend on the type of exfoliant and the strength of exfoliation desired. Surfactants that are useful, include but are not limited to DEA-oleth-3 phosphate, oleth-3, oleth-5, choleth-24, ceteth-24, and the like. Preservatives employed, may be in an amount of from about 0.01 to about 2.00 percent, preferably from about 0.02 to about 1.50 percent, of the formula weight. Examples of suitable preservatives are BHA, BHT, phenoxyethanol, ethyl paraben, propyl paraben, butyl paraben, or methyl paraben or an isomer, homolog, analog or derivative thereof.

The compositions of the invention are applied to the skin in a manner appropriate to the intended end result. For example, for the general promotion of the appearance of young, healthy skin by maintenance of the protective barrier of the stratum corneum, the best results are achieved after regular application over a period of time. A preferred method of obtaining the benefits of the composition is via chronic topical application of a safe and effective amount of a composition containing a mixture of cholesterol sulfate and the exfoliant. It is suggested as an example that topical application of the composition, in an amount of from about 0.1 mg/cm² to 2 mg/cm² of skin, be performed from about once per week to about 4 or 5 times daily, preferably from about 3 times a week to about 3 times daily, most preferably about once or twice per day. By "chronic" application, it is meant herein that the period of topical application may be over the lifetime of the user, preferably for a period of at least about one month, more preferably from about three months to about twenty years, more preferably from about six months to about ten years, more preferably still from about one year to about five years, thereby resulting in the treatment or prevention of damage to the skin experiencing a reduction or loss in barrier function.

When the composition is used in conjunction with a sunscreen, it is applied in the same amounts as specified above, on an as-needed basis, to mitigate the effects of exposure to the sun. When used in combination with a self-tanner, the composition is also applied in similar amounts, on the portion of the skin to be tanned, with repetition, again, on an as-needed basis.

The invention is further illustrated by the following non-limiting examples:

### Example I

This example illustrates the ability of cholesterol sulfate and an exfoliant to maintain protective barrier of the stratum corneum.

| **Ingredient** | **Product 1** |
|---|---|
| Surfactant | 1.10 |
| Propylene glycol | 0.20 |
| Squalane | 0.50 |
| BHT | 0.10 |
| Sclareolide | 0.10 |
| Cholesterol | 0.20 |
| Potassium Cholesterol sulfate | 0.20 |
| White birch | 0.20 |
| Butylene glycol | 1.00 |
| Chamomile | 0.03 |
| Bisabolol | 0.10 |
| Water | 64.31 |
| Carbowax PEG 3350 | 4.00 |
| Dimethicone copolyol | 2.00 |
| Glycereth-26 | 1.00 |
| Glucam-E20 | 4.00 |
| Methylparaben | 0.30 |
| Trisodium EDTA | 0.10 |
| Allantoin | 0.10 |
| Keltrol | 7.50 |
| Carbopol 981 | 11.00 |
| N-acetyl-D-glucosamine | 0.20 |
| Triethanolamine | 0.45 |
| Phenoxyethanol | 0.70 |
| Benzyl alcohol | 0.10 |
| Wheat bran extract | 0.10 |
| Olive extract | 0.10 |
| Linoleic and | 0.20 |
| Pigments | 0.11 |

The compositions are prepared according to the formula above and tested for their ability to repair the skin barrier after physical insult. The barrier condition is evaluated by challenging the skin with a tape strip and measuring the Trans Epidermal Water Loss (TEWL) for 3 days. Thirty female participants are used in the study. The participants have normal skin, are in good general health, and are free of any dermatological disorders. The participants apply the composition to one side of the face 2 times a day and the other side of the face is the untreated control. Their skin barrier is challenged by a method of tape stripping. The participants are acclimated to the test room conditions of about 40 percent relative humidity, and 70° F for about 15 to 20 minutes. An area is marked on the lower right cheek about 5 x 1 cm² near the jaw line and initial water evaporation measurements are taken in 3 separate spots about 1 cm apart in a row. Cello-tape, about 5 cm, is placed on the skin in the outlined area, starting from the top of the cheek. After one firm stroke is applied in each direction, the tap is removed by gently pulling in a downward direction parallel to the skin. This procedure is repeated and water evaporation is measured after every 5 strips until the barrier is disrupted as indicated by a minimum of 28 g/m² hr on one of the 3 spots. Both sides of the face are stripped in the same way. The participants return for TEWL evaluation 1, 2, and 3 days after tape stripping of the skin to monitor the repair of the skin barrier.

Barrier repair is the increase in the recovery of the skin on the stripped and treated side of the face compared with the stripped untreated side of the face. The total barrier repair over 3 days is calculated by determining the change in the area parameter. A small area indicates fast reparation of the skin barrier. The percent change in the area between the treated side of the face and the untreated side of the face is the difference in the total repair. The composition applied to the skin results in an 88 percent barrier repair over a placebo.

## Claims

1. A composition for topical application to the skin comprising a mixture of effective amounts of cholesterol sulfate or salts thereof, and an exfoliant.

2. The composition of claim 1 wherein the cholesterol sulfate or salts thereof is present in an amount between about 0.05 to about 5.00 percent, and the exfoliant is present in an amount between 0.1 to about 10.0 percent.

3. The composition of claim 1 wherein the composition contains a salt of cholesterol sulfate.

4. The composition of claim 3 wherein the salt is potassium.

5. The composition of claim 1 wherein the exfoliant is an amino sugar selected from the group consisting of N-acetyl-D-glucosamine, N-acetylgalactosamine, and a combination thereof.

6. The composition of claim 1 further comprising at least one fatty acid selected from the group consisting of butyric acid, caproic acid, octanoic acid, decanoic acid, dodecanoic acid, tetradecanoic acid, palmitic acid, stearic acid, linoleic acid and oleic acid.

7. The composition of claim 6 wherein said fatty acid is linoleic acid.

8. The composition of claim 1 further comprising cholesterol.

9. The composition of claim 1 further comprising both linoleic acid and cholesterol.

10. The composition of claim 1 further comprising sclareolide.

11. The composition of claim 1 further comprising a protease inhibitor selected from the group consisting of white birch extract, silver birch extract, Boswellia extract, bearberry extract, Centella asiatica extract, and Pygeum africanum extract.

12. The composition of claim 1 further comprising both sclareolide and white birch.

13. A cosmetic or pharmaceutical formulation for topical application of a composition to the skin, the formulation containing a mixture comprising cholesterol sulfate or salts thereof in an amount from about 0.05 to about 5.00 percent, and from about 0.1 to about 10.0 percent by weight of an amino sugar selected from the group consisting of N-acetyl-D-glucosamine, N-acetylgalactosamine, and a combination thereof by weight of the composition.

14. The formulation of claim 13 further comprising both cholesterol and a fatty acid selected from the group consisting of butyric acid, caproic acid, octanoic acid, decanoic acid, dodecanoic acid, tetradecanoic acid, palmitic acid, stearic acid, linoleic acid and oleic acid.

15. The formulation of claim 14 wherein the fatty acid is linoleic acid present in an amount less than 1 percent and the cholesterol is present in an amount less than 1 percent.

16. Use of an effective amount of a mixture comprising cholesterol sulfate or salts thereof in an amount from about 0.05 to about 5.00 percent by weight of the composition, and from about 0.1 to 10.0 percent by weight of an amino sugar selected from the group consisting of N-acetyl-D-glucosamine, Nacetylgalactosamine, and a combination thereof, for preparing a composition for application to the skin for improving or maintaining a healthy skin barrier.

17. The use of claim 16 in which the mixture comprises from about 0.1 to about 2.0 percent cholesterol sulfate.

18. The use of claim 16 in which the composition comprises about 0.04 to about 1.00 percent cholesterol sulfate.

19. Use of an effective amount of a mixture comprising cholesterol sulfate or salts thereof in an amount from about 0.05 to about 5.00 percent, and about 0.1 to about 10.0 percent of an amino sugar selected from the group consisting of N-acetyl-D-glucosamine, N-acetylgalactosamine, and a combination thereof by weight of the composition for preparing a composition for application to the skin for treating or preventing damage to the skin, wherein the damage is associated with a reduction or loss of skin barrier function.

20. The use of claim 19 further comprising cholesterol sulfate or salts thereof in an amount of about 0.1 to 2.0 percent, about 0.5 to 8.0 percent of N-acetyl-D-glucosamine, cholesterol in an amount of about 0.2 to 1.0 percent, linoleic acid in an amount of about 0.2 to 1.0 percent by weight of the of the composition, sclareolide in an amount of about 0.001 to about 1.000 percent, and white birch in an amount of about 0.001 to about 1.000 percent.

## Patentansprüche

1. Zusammensetzung zur örtlichen Anwendung auf der Haut, welche ein Gemisch aus wirksamen Mengen von Cholesterolsulfat oder dessen Salzen und einem Abschuppungsmittel umfasst.

2. Zusammensetzung nach Anspruch 1, wobei das Cholesterolsulfat oder dessen Salze in einer Menge von etwa 0,05 bis etwa 5,00 Prozent vorliegen und das Abschuppungsmittel in einer Menge von 0,1 bis etwa 10,0 Prozent vorliegt.

3. Zusammensetzung nach Anspruch 1, wobei die Zusammensetzung ein Salz des Cholesterolsulfats enthält.

4. Zusammensetzung nach Anspruch 3, wobei es sich bei dem Salz um ein Kaliumsalz handelt.

5. Zusammensetzung nach Anspruch 1, wobei es sich bei dem Abschuppungsmittel um einen Aminozucker handelt, ausgewählt aus der Gruppe, bestehend aus N-Acetyl-D-glucosamin, N-Acetylgalactosamin und einer Kombination daraus.

6. Zusammensetzung nach Anspruch 1, welche ferner mindestens eine Fettsäure umfasst, ausgewählt aus der Gruppe, bestehend aus Buttersäure, Hexansäure, Octansäure, Decansäure, Dodecansäure, Tetradecansäure, Palmitinsäure, Stearinsäure, Linolsäure und Ölsäure.

7. Zusammensetzung nach Anspruch 6, wobei es sich bei der Fettsäure um Linolsäure handelt.

8. Zusammensetzung nach Anspruch 1, welche ferner Cholesterol umfasst.

9. Zusammensetzung nach Anspruch 1, welche ferner sowohl Linolsäure als auch Cholesterol umfasst.

10. Zusammensetzung nach Anspruch 1, welche ferner Sclareolid umfasst.

11. Zusammensetzung nach Anspruch 1, welche ferner einen Proteasehemmer umfasst, ausgewählt aus der Gruppe, bestehend aus Papierbirkenextrakt, Weißbirkenextrakt, Boswelliaextrakt, Bärentraubenextrakt, Centella-asiatica-Extrakt und Pygeum-africanum-Extrakt.

12. Zusammensetzung nach Anspruch 1, welche ferner sowohl Sclareolid als auch Papierbirke umfasst.

13. Kosmetische oder pharmazeutische Formulierung zur örtlichen Anwendung einer Zusammensetzung auf der Haut, wobei die Formulierung ein Gemisch enthält, welches Cholesterolsulfat oder dessen Salze in einer Menge von etwa 0,05 bis etwa 5,00 Prozent und einen Aminozucker in einer Menge von etwa 0,1 bis etwa 10,0 Gewichtsprozent der Zusammensetzung umfasst, wobei der Aminozucker ausgewählt ist aus der Gruppe, bestehend aus aus N-Acetyl-D-glucosamin, N-Acetylgalactosamin und einer Kombination daraus.

14. Formulierung nach Anspruch 13, welche ferner sowohl Cholesterol als auch eine Fettsäure umfasst, ausgewählt aus der Gruppe, bestehend aus Buttersäure, Hexansäure, Octansäure, Decansäure, Dodecansäure, Tetradecansäure, Palmitinsäure, Stearinsäure, Linolsäure und Ölsäure.

15. Formulierung nach Anspruch 14, wobei es sich bei der Fettsäure um Linolsäure handelt, die in einer Menge von weniger als 1 Prozent vorliegt, und wobei das Cholesterol in einer Menge von weniger als 1 Prozent vorliegt.

16. Verwendung einer wirksamen Menge eines Gemisches, welches Cholesterolsulfat oder dessen Salze in einer Menge von etwa 0,05 bis etwa 5,00 Gewichtsprozent der Zusammensetzung und einen Aminozucker in einer Menge von etwa 0,1 bis etwa 10,0 Gewichtsprozent umfasst, wobei der Aminozucker ausgewählt ist aus der Gruppe, bestehend aus aus N-Acetyl-D-glucosamin, N-Acetylgalactosamin und einer Kombination daraus, zur Herstellung einer Zusammensetzung zur Anwendung auf der Haut zur Verbesserung oder Bewahrung einer gesunden Hautbarriere.

17. Verwendung nach Anspruch 16, bei welcher das Gemisch von etwa 0,1 bis etwa 2,0 Prozent Cholesterolsulfat umfasst.

18. Verwendung nach Anspruch 16, bei welcher die Zusammensetzung von etwa 0,04 bis etwa 1,00 Prozent Cholesterolsulfat umfasst.

19. Verwendung einer wirksamen Menge eines Gemisches, welches Cholesterolsulfat oder dessen Salze in einer Menge von etwa 0,05 bis etwa 5,00 Prozent und einen Aminozucker in einer Menge von etwa 0,1 bis etwa 10,0 Gewichtsprozent der Zusammensetzung umfasst, wobei der Aminozucker ausgewählt ist aus der Gruppe, bestehend aus aus N-Acetyl-D-glucosamin, N-Acetylgalactosamin und einer Kombination daraus, zur Herstellung einer Zusammensetzung zur Anwendung auf der Haut zur Behandlung oder Prävention einer Hautschädigung, wobei die Schädigung mit einer Verringerung oder einem Verlust der Hautbarrierenfunktion in Zusammenhang steht.

20. Verwendung nach Anspruch 19, wobei das Gemisch Cholesterolsulfat oder dessen Salze in einer Menge von etwa 0,1 bis 2,0 Gewichtsprozent, N-Acetyl-D-glucosamin in einer Menge von etwa 0,5 bis 8,0 Gewichtsprozent, Cholesterol in einer Menge von etwa 0,2 bis 1,0 Gewichtsprozent, Linolsäure in einer Menge von etwa 0,2 bis 1,0 Gewichtsprozent der Zusammensetzung, Sclareolid in einer Menge von etwa 0,001 bis etwa 1,000 Prozent und Papierbirke in einer Menge von etwa 0,001 bis 1,000 Prozent umfasst.

## Revendications

1. Une composition pour application topique à la peau comprenant un mélange de quantités efficaces de sulfate de cholestérol, ou de sels en dérivant, et d'un exfoliant.

2. La composition selon la revendication 1, dans laquelle le sulfate de cholestérol ou les sels en dérivant sont présents en une proportion comprise entre environ 0,05% et environ 5,00%, et l'exfoliant est présent en une proportion comprise entre environ 0, 1 % et environ 10,0%.

3. La composition selon la revendication 1, dans laquelle la composition contient un sel de sulfate de cholestérol.

4. La composition selon la revendication 3, dans laquelle le sel est le sel de potassium.

5. La composition selon la revendication 1, dans laquelle l'exfoliant est un aminosucre choisi dans le groupe consistant en la N-acétyl-D-glucosamine, la N-acétylgalactos-amine et leurs combinaisons.

6. La composition selon la revendication 1, comprenant en outre au moins un acide gras choisi dans le groupe comprenant l'acide butyrique, l'acide caproïque, l'acide octanoïque, l'acide décanoïque, l'acide dodécanoïque, l'acide tetradécanoïque, l'acide palmitique, l'acide stéarique, l'acide linoléique et l'acide oléique.

7. La composition selon la revendication 6, dans laquelle ledit acide gras est l'acide linoléique.

8. La composition selon la revendication 1, comprenant en outre du cholestérol.

9. La composition selon la revendication 1, comprenant en outre tant de l'acide linoléique que du cholestérol.

10. La composition selon la revendication 1, comprenant en outre de la sc 1 aréolide.

11. La composition selon la revendication 1 comprenant en outre un inhibiteur de protéase choisi dans le groupe comprenant les extraits de bouleau blanc, extraits de bouleau argenté, extraits de Boswellia, extraits de busserole, extraits de Centella asiatica et extraits de Pygeum africanum.

12. La composition selon la revendication 1 comprenant en outre de la sclaréolide et du bouleau blanc.

13. Une formulation cosmétique ou pharmaceutique pour l'application topique d'une composition à la peau, ladite formulation contenant un mélange comprenant du sulfate de cholestérol, ou des sels en dérivant, en une proportion, exprimée par rapport au poids de ladite composition, comprise entre environ 0,05% et environ 5,00%, et contenant entre environ 0,1% et environ 10,0% en poids d'un aminosucre choisi dans le groupe comprenant la N-acétyl-D-glucosamine, la N-acétylgalactosamine et leurs combinaisons.

14. La formulation selon la revendication 13 comprenant en outre du cholestérol et un acide gras choisi dans le groupe comprenant l'acide butyrique, l'acide caproïque, l'acide octanoïque, l'acide décanoïque, l'acide dodécanoïque, l'acide tetradécanoïque, l'acide palmitique, l'acide stéarique, l'acide linoléique et l'acide oléique.

15. La formulation selon la revendication 14, dans laquelle l'acide gras est l'acide linoléique présent en une proportion inférieure à 1% et dans laquelle le cholestérol est présent en une proportion inférieure à 1 %.

16. Utilisation d'une quantité efficace d'un mélange comprenant du sulfate de cholestérol, ou des sels en dérivant, en une proportion, exprimée par rapport au poids de ladite composition, comprise entre environ 0,05% et environ 5,00%, et comprenant un aminosucre choisi dans le groupe comprenant la N-acétyl-D-glucosamine, la N-acétylgalactosamine et leurs combinaisons en une proportion comprise entre environ 0,1% et environ 10,0% en poids, pour la préparation d'une composition pour application à la peau pour l'amélioration ou le maintien d'une barrière cutanée saine

17. L'utilisation selon la revendication 16, dans laquelle le mélange comprend du sulfate de cholestérol en une proportion comprise entre environ 0,1% et environ 2,0%.

18. L'utilisation selon la revendication 16, dans laquelle la composition comprend du sulfate de cholestérol en une proportion comprise entre 0,04% et environ 1,00%.

19. Utilisation d'une quantité efficace d'un mélange comprenant du sulfate de cholestérol, ou des sels en dérivant, en une proportion, exprimée par rapport au poids de ladite composition, comprise entre environ 0,05% et environ 5,00%, et comprenant un aminosucre choisi dans le groupe comprenant la N-acétyl-D-glucosamine, la N-acétylgalactosamine et leurs combinaisons en une proportion comprise entre environ 0,1% et environ 10,0% en poids, pour la préparation d'une composition pour application à la peau pour traiter ou prévenir des dommages occasionnés à la peau, utilisation dans laquelle ledit dommage consiste en une réduction ou une perte de la fonction de barrière cutanée.

20. L'utilisation selon la revendication 19, comprenant en outre, exprimés par rapport au poids de ladite composition, du sulfate de cholestérol, ou des sels en dérivant, en une proportion comprise entre 0,1% et 2,0%, de la N-acétyl-Dglucosamine en une proportion comprise entre environ 0,5% et 8,0%, du cholestérol en une proportion comprise entre environ 0,2% et 1,0%, de l'acide linoléique en une proportion comprise entre environ 0,2% et 1,0%, de la sclaréolide en une proportion comprise entre environ 0,001% et environ 1,000% et du bouleau blanc en une proportion comprise entre environ 0,001% et environ 1,000%.
